# EUROPEAN PATENT SPECIFICATION

(11) **EP 2 919 815 B1**
(45) Date of publication and mention of the grant of the patent: **06.09.2017**
(21) Application number: 13801824.7
(22) Date of filing: 12.11.2013
(51) Int. Cl.: A61K 45/06, A61K 9/14, A61K 31/403, A61K 31/404, A61K 31/44, A61K 31/4422, A61K 9/20, A61K 9/16, A61K 9/50

(54) **A PHARMACEUTICAL COMPOSITION CONTAINING AN ACE INHIBITOR AND A CALCIUM CHANNEL BLOCKER**
PHARMAZEUTISCHE ZUBEREITUNG ENTHALTEND EINEN ACE-HEMMER UND EINEN CALCIUMKANALBLOCKER
COMPOSITION PHARMACEUTIQUE COMPRENANT UN INHIBITEUR DE L'ACE ET UN INHIBITEUR DES CANAUX CALCIQUES

(30) Priority: 15.11.2012 PL 40163212
(43) Date of publication of application: 23.09.2015
(73) Proprietor: Adamed sp. z o.o., 05-152 Czosnów k/Warszawy (PL)
(72) Inventor: TURCZYN, Elzbieta, 32-005 Niepolomice (PL)
(74) Representative: Sitkowska, Jadwiga
(86) International application number: PCT/IB2013/060069
(87) International publication number: WO 2014/076632

(56) References cited:
- WO-A1-2005/120502
- WO-A1-2007/040511
- WO-A2-2006/097943
- WO-A2-2011/104588

## Description

### Field of the invention

The invention relates to a stable pharmaceutical composition comprising an angiotensin converting enzyme (ACE) inhibitor and a calcium channel blocker (CCB), a process for manufacturing of this composition, a unit dosage form containing this composition, and to the use of this composition in therapies related to hypertension.

### Prior art

Hypertension is one of the most serious health problems in the world. It is considered a major mortality risk factor and the most common risk factor for cardiovascular diseases.

Clinical trials have shown a limited efficacy of traditionally used monotherapy.

The use of a combination of two medicinal substances from different therapeutic groups acting by different pathophysiological mechanisms enables to achieve a better antihypertensive effect and more effective protection against organ damages observed in hypertension. In addition, the use of combination products is more effective than monotherapy because it is possible to simplify the treatment regimens and to increase patients compliance.

Angiotensin converting enzyme inhibitors, also known as ACE inhibitors or ACE-I, are effective in all types of hypertension. They act mainly by inhibiting the synthesis of angiotensin II and by blocking the degradation of bradykinin.

In practice, during the treatment of hypertension the most commonly used combinations are ACE inhibitors administered with other therapeutic agents such as calcium channel blockers (CCB/Ca⁺² antagonists), diuretics, HMG-CoA reductase inhibitors, beta-blockers, nonsteroidal anti-inflammatory drugs, angiotensin antagonists II, and combinations thereof.

Calcium channel blockers (CCB), which are used in combination with ACE inhibitors, are also known as calcium antagonists. They activate the renin-angiotensin-aldosterone system (RAAS) and sympathetic nervous system by vasodilatation. This, in turn, causes vasoconstriction and retention of sodium and water in the organism.

Combination of calcium antagonists therapy with the use of ACE inhibitors inhibits the adverse effects of compensatory mechanisms, as well as enhances the protective effect with respect to development of diabetic nephropathy and reduction of left ventricular hypertrophy.

Both ACE inhibitors and calcium channel blockers are susceptible to degradation. Prior art documents describe the problems associated with the lack of stability of compositions comprising both ACE inhibitors and calcium channel blockers.

Types and methods of identifying of the individual degradants of ACE inhibitors and calcium channel blockers are described in US Pharmacopoeia and European Pharmacopoeia monographs. The pharmaceutically acceptable limits for these degradants are specified, for example in the British Pharmacopoeia.

Decomposition of the active substances (ACE inhibitors and calcium channel blockers) during manufacture and storage may adversely affect the effectiveness of the combined therapeutic and may result in that the resulting medicinal product fails to meet the requirements of chemical purity or pharmaceutical potency.

In the case of calcium channel blockers degradation occurs primarily in the presence of water and alcohols, leading to hydrolysis, transesterification or oxidation.

In the case of ACE inhibitors degradation occurs most frequently in contact with water or under elevated temperature, leading to the esterification, dehydration or hydrolysis (due to the intramolecular cyclization with the elimination of water). The adverse effect of excipients and mechanical stress (especially compression) on the stability of certain ACE inhibitors is also described.

Solutions to the problems related to the stability of ACE inhibitors are exemplified in US5151433, where stabilizing coating of an ACE inhibitor ramipril was applied. EP 317878 describes that mechanical treatment contributes to the increase of the percentage of ramipril impurities and to decrease of its contents in the pharmaceutical composition. US20030215526 discloses the use of an alkaline substance for stabilization of ACE inhibitors, and US2005181055 the use of a basic substance and polyvinylpyrrolidone. Pharmaceutical compositions which contain an alkali metal carbonate or alkaline earth metal as stabilizers of ACE inhibitors are also described in the prior art (EP 280999, W2005094793 and WO2003059388).

Increase of ACE inhibitors degradants is also observed in the case of a fixed dosage form containing both an ACE inhibitor and another active ingredient, especially in case of different chemical properties of the active ingredients of such a composition. Such a pharmaceutical incompatibility implies the need of stabilization of the components of the composition. From the prior art it is known that such stabilization can be achieved by the use of stabilizing substances that affect the active ingredients, or by their physical separation.

Application BR 00/03282 discloses a composition containing ramipril and amlodipine physically separated from each other. The composition is in the form of a capsule containing two active ingredients in the form of coated granules or coated tablets. Physical separation is also obtained when the composition is in the form of a coated tablet, in which amlodipine and ramipril are separated by an interior layer.

As described in EP 1948136, the effect of stabilization of angiotensin-converting enzyme inhibitors sensitive to the influence of another active substance can also be achieved by coating with glyceryl dibehenate.

WO2008065485 discloses a stable composition in the form of a capsule containing a granulate of benazepril and a blend of amlodipine, wherein the active ingredients are not physically separated. The composition contains alkaline agents, by which pH> 6 is obtained, and as a consequence ACE inhibitor which is sensitive to low pH, is stabilized.

WO2011/104588 discloses pharmaceutical compositions in the form of a tablet comprising ramipril and amlodipine besylate with pharmaceutically acceptable excipients, wherein ramipril is in the form of granules, said coated granules being embedded in an extragranular matrix comprising amlodipine besylate. Disadvantages of capsule forms and superiority of tablet incorporating ramipril coated granules and amlodipine powder over capsules is taught.

Also known is another pharmaceutical composition in the form of a capsule containing granulated ACE inhibitor and amlodipine, wherein the active ingredients are not physically separated from each other. In such a composition disclosed in WO2007040511 among others lactose monohydrate and polysorbate 80 were used. Stabilizing properties of lactose monohydrate against instable ACE inhibitors is described in WO2003028707. The use of polysorbate 80 is also known in such compositions to improve the impurity profile of the active substances that are sensitive or chemically unstable.

Compositions of captopril/nifedipine in a form of simple blend compressed further to a tablet and composition of mixture of enalapril/nitredipine filled into a capsule are disclosed in EP0381075. The active substances are not separated and mechanical stress is not applied (in the case of capsule). Data concerning impurities level of these compositions are not provided. However, as shown by stability experiments performed by the present applicant, simple blends of ACE-I/CCB (neither stabilized nor separated) do not provide pharmaceutically acceptable results.

A stable pharmaceutical composition comprising an ACE inhibitor and a calcium channel blocker prepared with the use of physical separation of the components is costly and labor intensive. Providing a suitable pH for the stabilization of ACE inhibitor is in turn disadvantageous for the stability of calcium channel blocker. Similarly, the use of stabilizers of ACE inhibitor can lead to the increase of the calcium channel blocker degradants.

Similarly, mechanical stress of ACE inhibitors applied during the operations of the physical separation of the active substances from each other or during the aggregation of the fraction containing the ACE inhibitor (e.g. granulation) may contribute to the increase of impurities level.

It is therefore desirable to increase the stability of a pharmaceutical composition comprising an ACE inhibitor and a calcium channel blocker without the use of stabilizing agents and pH adjusting agents, while minimizing the mechanical stress of an ACE inhibitor. In order to optimize the process of the preparation of such a composition, the absence of physical separation of the active ingredients in is also desired.

The above problems are overcome by the present invention, which provides a stable pharmaceutical composition comprising an ACE inhibitor and a calcium channel blocker, wherein the level of degradants of the active substances is maintained at a pharmaceutically acceptable level without the need to use stabilizing agents and pH adjusting agents, and wherein the mechanical treatment of ACE inhibitor is minimized. The invention also provides a pharmaceutical composition, wherein the active substances, i.e. the ACE inhibitor and calcium channel blocker, are not physically separated from each other.

A pharmaceutical composition, which is an object of the invention, is the blend of:
a) a powder containing an ACE inhibitor wherein the ACE inhibitor is selected from ramipril, perindopril and pharmaceutically acceptable salts, solvates and hydrates thereof, and
b) a granulate containing a calcium channel blocker wherein the calcium channel blocker is selected from amlodipine and pharmaceutically acceptable salts, solvates and hydrates thereof,
wherein the powder a) and the granulate b) contain one or more excipients, and wherein the powder a) is not physically separated from the granulate b).

There is also described a process of the preparation of a pharmaceutical composition of the invention.

Furthermore, the invention provides a unit dosage form comprising a pharmaceutical composition of the invention.

### Description of the drawings

Fig.1 is a flowchart illustrating a process of the preparation of the composition according to the invention.
Fig. 2 presents the results of assessment of the compatibility of active ingredients amlodipine benzenesulfonate and perindopril tert-butylamine by DSC analysis.

### Detailed Description of the Invention

The meaning of the terms used in the description is explained below.

The term "stable pharmaceutical composition" refers to a composition which is not susceptible to degradation and in which the level of all active substances degradants is maintained at a pharmacologically acceptable level-that is the level specified in the relevant monographs of medicinal substances of British Pharmacopoeia or the United States Pharmacopoeia, and in the case of absence of an appropriate monograph experimentally determined on the basis of the stability tests in accordance with the guidelines of the International Conference on Harmonisation Requirements for Medicines.

The term "without physical separation" means that active substances are in physical contact with each other. Remaining of the active substances in contact means that during technological process no techniques leading to physical separation are used, such as, for example, coating of the granules, grains or pellets containing the active substances prior to their incorporation in a capsule, sachet or their use as components of solid pharmaceutical forms, application of separating layers in solid dosage forms, microencapsulation, nanoencapsulation, the formation of microspheres, the use of two-chamber therapeutic systems with membranes semipermeable for water or use of a matrix (array) serving as a carrier for the drug in separate chambers, for example a gel matrix.

The term "calcium channel blocker" refers to substances which are capable of blocking (inhibition) of calcium channel in cells of the body. Calcium channel is a type of membrane receptor and thus may bind with specific substances, called antagonists, thereby blocking or reducing the possibility of opening the channels. Lowering the ability to open calcium channels reduces the influx of calcium ions into cells and decreases their intracellular concentration. The term "calcium channel blocker" is synonymous with the term "calcium antagonist" and "calcium channel antagonist".

The term "ACE inhibitor" is synonymous with the term ACE-I and describes an angiotensin converting enzyme inhibitor, i.e. an active substance acting mainly by inhibiting the synthesis of angiotensin II and by blocking the degradation of bradykinin.

The term "uniform mixture" relates to a homogeneous mixture, i.e. a mixture which is characterized by substantially the same distribution of components in a given sample throughout the whole weight or volume of the mixture.

The object of the invention is a stable pharmaceutical composition consisting of the blend of
a) a powder containing an ACE inhibitor selected from ramipril, perindopril and pharmaceutically acceptable salts, solvates and hydrates thereof, and
b) a granulate containing a calcium channel blocker selected from amlodipine and pharmaceutically acceptable salts, solvates and hydrates thereof,
wherein the powder a) and the granulate b) contain one or more excipients and wherein the powder a) is not physically separated from the granulate b).

In a preferred embodiment, the stable pharmaceutical composition of the invention comprises a salt of perindopril with tert-butylamine or arginine, or ramipril in the form of free acid as the ACE inhibitor.

In one embodiment, the composition of the invention comprises the ACE inhibitor in a percentage from 2.5 to 14% by weight, with respect to the total weight of the powder a).

In a preferred embodiment, a calcium channel blocker is amlodipine benzenesulfonate.

In one embodiment, the composition of the invention comprises the calcium channel blocker in a percentage from 3.5 to 11% by weight, with respect to the total weight of the granulate b).

A composition as described in all the above variants, in addition to the ACE inhibitor and the calcium channel blocker may contain one or more excipients or carriers selected from fillers (diluents), binders, disintegrants, adsorbents, absorbents, colorants, sweeteners , glidants, etc.

In one embodiment, the composition of the invention contains a pharmaceutically acceptable excipient selected from fillers, binders, disintegrants and glidants.

Fillers that are useful in the composition as described in all the above embodiments include starch, microcrystalline cellulose, microcrystalline cellulose with silica, sugars such as sucrose, dextrose, and polyhydric alcohols such as mannitol and sorbitol, and other known fillers.

Glidants that are useful in the composition as described in all the above embodiments include sodium stearyl fumarate, stearic acid, metal stearates such as magnesium, calcium or zinc, talc, sodium lauryl sulfate, magnesium lauryl sulfate, hydrogenated vegetable oil, hydrogenated castor oil, glycerol dibehenate, glycerol palmitostearate, glycerol monostearate, polyethylene glycols, polymers of ethylene oxide, silicon dioxide, mineral oil, D-leucine and other known lubricants.

Binders that are useful in the composition as described in all the above embodiments include cellulose derivatives such as hydroxypropylmethylcellulose, hydroxypropylcellulose, ethyl cellulose, hydroxyethylcellulose, methylcellulose, gelatin, shellac, zein, polymethacrylates, polyvinylpyrrolidone, starch, pregelatinized starch, gum, alginic acids and salts thereof such as sodium alginate, anhydrous or hydrated dibasic calcium phosphate, tribasic calcium phosphate, synthetic resins, magnesium aluminum silicate, polyethylene glycol, dextrose, xylitol, and bentonites.

Disintegrants that are useful in the composition as described in all the above embodiments include sodium starch glycolate, cross-linked sodium carboxymethylcellulose, hydroxyethylcellulose, sodium carboxymethylcellulose, calcium carboxymethylcellulose, starch, pregelatinized starch, crospovidone, microcrystalline cellulose and effervescent mixtures, and other excipients known for example from "Handbook of Pharmaceutical Excipients" edited by R. C. Rowe, P. J. Sheskey i S. Owen, Pharmaceutical Press, edition VII.

In one embodiment of the composition of the invention the powder a) comprises excipients selected from a filler and a glidant.

In a preferred embodiment of the composition according to the invention the percentage of the filler in the powder a) is in a range from 0 to 97% by weight, and the percentage of the glidant is in a range from 0 to 2.5% by weight, with respect to the total weight of the powder a).

Preferably, the powder a) contains from 80 to 97% by weight of the filler. Particularly preferred filler in the powder a) is pregelatinized starch. Preferably, powder a) contains from 0.5 to 2.5% by weight of a glidant.

Particularly preferably, the glidant in the powder a) is sodium stearyl fumarate.

In one embodiment of the composition according to the invention the granulate b) contains excipients selected from a filler, a binder, a disintegrant and a glidant.

In a preferred embodiment of the composition according to the invention the percentage of a filler in the granulate b) is from 0 to 70% by weight, the percentage of a binder is from 0 to 30% by weight, the percentage of a disintegrant is from 0 to 4.3% by weight, and the percentage of a glidant is from 0 to 1.5% by weight, with respect to the total weight of the granulate b). Preferably, the granulate b) contains from 55 to 70% by weight of a filler. A preferred filler in the granulate b) is microcrystalline cellulose. Preferably, the granulate b) contains from 20 to 30% by weight of a binder.

Particularly preferably, the binder in the granulate b) is anhydrous dibasic calcium phosphate, hydroxypropylmethylcellulose, or a mixture thereof.

Preferably, the granulate b) contains from 0.8 to 4.3% by weight of a disintegrant.

Particularly preferred disintegrant in the granulate b) is sodium starch glycolate.

Preferably, the granulate b) contains from 0.8 to 1.5% by weight of a glidant.

Particularly preferably, the glidant in the granulate b) is sodium stearyl fumarate. The powder a) is not physically separated from the granules b) in the composition of the invention. In this variant, the active compounds such as ACE inhibitors and calcium channel blockers are not physically separated from each other.

However, in one embodiment of the composition according to the invention, the granulate b) is coated. The aim of the coating is to produce a coat of a granule. Coating allows the physical separation of the active substances from each other. The main component of the coat is a film-forming polymer. Such polymer can include, e.g. cellulose derivatives as hypromellose, methylcellulose, ethylcellulose, polyvinylpyrrolidone, polyvinyl alcohol, polymethacrylates and other substances known to those skilled in the art.

Additionally, substances preventing sticking the granules can be used, such as colloidal silica, talc and other substances known in the art. Additionally, other substances well-known in the art to improve the quality of the coating can be used. The percentage of the coating relative to the weight of the uncoated granulate is selected in accordance with principles known in the art, for example according to the textbook "Farmacja stosowana " edited by S. Janicki, A. Fiebig, PZWL, 4^{th} ed. Preferably, the percentage of a coat is from 4-10% by weight with respect to the weight of the uncoated granulate.

Preferably, the excipients used in all embodiments of the composition of the invention do not exert a stabilizing effect on the active substances.

The pH of the stable pharmaceutical composition of the invention comprising an ACE inhibitor in the form of a powder, a calcium channel blocker in the granular form, and pharmaceutically acceptable filler is from 5.1 to 5.6.

In a preferred embodiment, the pH of the powder a) containing an ACE inhibitor is in a range from 4.8 to 5.1.

In none of the embodiments of the invention pH adjustment is performed.

There is also disclosed a process for the preparation of a composition according to the invention.

The process comprises:
- preparation of a powder blend containing an ACE inhibitor and excipients,
- preparation of a granulate containing a calcium channel blocker, and
- blending the prepared granulate with the prepared powder to form a uniform blend.

In one embodiment, preparation of the granulate containing the calcium channel blocker comprises sieving of the active substance with excipients, blending, compacting and homogenization, while preparation of the powder comprises sieving the ACE inhibitor with excipients and blending.

In one embodiment, the calcium channel blocker is sieved, blended and compacted with fillers, binders, disintegrants and glidants, while the ACE inhibitor is sieved and blended with fillers and glidants.

In another embodiment, the calcium channel blocker is blended and compacted with microcrystalline cellulose, sodium starch glycolate, sodium stearyl fumarate and dibasic calcium phosphate, while the ACE inhibitor is sieved and blended with Pregelatinized starch and sodium stearyl fumarate.

In another embodiment, the calcium channel blocker is blended with microcrystalline cellulose, sodium starch glycolate, sodium stearyl fumarate and dibasic calcium phosphate, slugged, and then screened on sieves to form granules. However, this embodiment is less favourable and more labor-intensive.

In another embodiment, the blend of a calcium channel blocker and fillers is subjected to wet granulation, using a suitable substance to allow wet granulation, selected from water, isopropyl alcohol, ethanol, acetone, methylene chloride and their mixtures, hydrophilic substances having binding properties, such as 2-20% aqueous or ethanol solutions of gelatin, aqueous solutions of glucose, sucrose or sorbitol at different concentrations, corn syrup, solutions of cellulose derivatives (hydroxypropylcellulose, hydroxypropylmethylcellulose, methylcellulose, carboxymethylcellulose, hydroxyethylcellulose and hydroxyethylcellulose), 1% sodium alginate solution, a solution of polyvinyl alcohol, a solution of polyvinylpyrrolidone, and other excipients known from the art used in the granulation process as described for example in the textbook " Farmacja stosowana " edited by S. Janicki, A. Fiebig, PZWL, 4th ed.

The remaining steps in the process of the preparation of a composition of the invention containing granulate formed by wet granulation remain unchanged.

It has been stated by the applicant that when the powder a) containing an ACE inhibitor is further subjected to granulation similarly as calcium channel blocker, whether by dry compaction or wet granulation using excipients, final composition obtained by blending two granulates is less favorable than the blend of the invention.

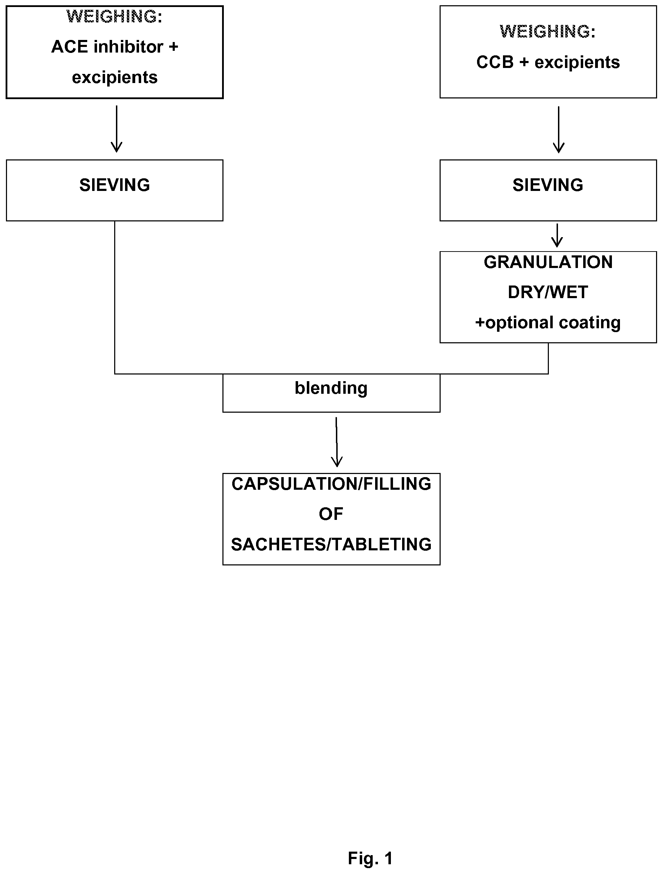

The process of the preparation of the composition of the invention is illustrated on a flow chart shown in Fig. 1 and described in details in Examples.

The stable composition as described in all of the above embodiments containing the powder a) of an ACE inhibitor, and granulate b) of the calcium channel blocker can be used as:
- an oral powder or the component of an oral powder,
- a composition for preparing an oral solution or suspension, or syrup.

In a further aspect, the invention also comprises a unit dosage form containing a composition as described in all of the above embodiments. In a preferred embodiment, the unit dosage form containing a stable composition consisting of the powder a) of an ACE inhibitor and the granulate b) of the calcium channel blocker is selected from:
- pellets or granules,
- tablets,
- sachetes, including sticks
- ampoules,
- elastic (soft) or hard gelatin capsules,
- starch capsules, and
- hydroxypropylmethylcellulose capsules,
wherein the pellets, granules, tablets and capsules can be destined for immediate release or modified release, for example, enteric-coated.

In one variant of the above aspect the unit dosage form contains an ACE inhibitor in an amount of 0.9 to 5.0% by weight and a calcium channel blocker in an amount of 2.6 to 7.1% by weight, with respect to the total weight of the composition.

Preparation of an oral solution, oral suspension, syrup, pellets, granules, tablets, sachets, sticks, ampoules, elastic (soft) or hard gelatin capsule, starch capsule, and hydroxypropylmethylcellulose capsules with the use of the composition according to the invention is carried out using standard methods and means known in the art and described for example in the textbook "Farmacja stosowana " edited by S. Janicki, A. Fiebig, PZWL, 4th ed.

The composition according to the invention or prepared by the process according to the invention is stable during 3 and 6 months storage at 40 °C and 75% relative humidity. Increase in total impurities of an ACE inhibitor and calcium channel blocker during storage does not exceed the limits specified for pharmaceutical compositions.

The dosage schedule of the composition according to the invention varies depending on sex, age and weight of the patient, the nature of the disease, and optionally other treatments to which the patient is subjected. The dose of a compound of an ACE inhibitor may be lower than the dose used when it is administered alone. A suitable route of administration of the composition is in particular the oral route.

The invention will be described in the following examples of specific compositions and processes for the preparation of the compositions and of the dosage forms:
Generally, preparation of the composition of the present invention comprises weighing the active substances and excipients in the amounts shown in Table 1 and Table 2 (for dry granulation), and Table 3 (for wet granulation) appropriately for the desired dosage, in a proportion appropriate to the size of the batch, and then following the manufacturing schedule shown in a flow-chart of Figure 1. In addition to the ingredients listed in Table 1, 2 and 3, a coating comprising hydroxypropylmethylcellulose and colloidal silica can be optionally used to coat granules.

Abbreviations used below have the following meanings:
AML - amlodipine benzenesulfonate
RAM - ramipril
PER - tert-butylamine salt of perindopril

**Table 1. Qualitative and quantitative composition [mg] of amlodipine/ramipril - dry granulation**

| component | AML/RAM 5/2.5 mg | AML/RAM 5/5 mg | AML/RAM 5/10 mg | AML/RAM 10/5 mg | AML/RAM 10/10 mg |
|---|---|---|---|---|---|
| Components of the powder | | | | | |
| ramipril | 2.50 | 5.00 | 10.00 | 5.00 | 10.0 |
| pregelatinized starch | 66.0 | 66.00 | 66.00 | 66.00 | 66.0 |
| Sodium stearyl fumarate | 1.50 | 1.50 | 1.50 | 1.50 | 1.50 |
| Weight of the powder | 70.00 | 72.50 | 77.50 | 72.50 | 77.50 |

| Components of the granulate | | | | | |
|---|---|---|---|---|---|
| amlodipine benzenesulfonate | 6.93 | 6.93 | 6.93 | 13.87 | 13.87 |
| microcrystalline cellulose | 95.47 | 95.47 | 95.47 | 88.53 | 88.53 |
| anhydrous dibasic calcium phosphate, | 37.00 | 37.00 | 37.00 | 37.00 | 37.00 |
| Sodium starch glycolate | 2.00 | 2.00 | 2.00 | 2.00 | 2.00 |
| sodium stearyl fumarate | 1.60 | 1.60 | 1.60 | 1.60 | 1.60 |
| Weight of the granulate | 143.00 | | | | |
| *Total weight of the blend* | 213.00 | 215.50 | 220.50 | 215.50 | 220.50 |

**Table 2. Qualitative and quantitative composition [mg] of amlodipine/perindopril - dry granulation**

| Component | AML/PER 5mg/4 mg | AML/PER 5mg/8 mg | AML/PER 10mg/4 mg | AML/PER 10mg/8 mg |
|---|---|---|---|---|
| Components of the powder | | | | |
| Tert-butylamine salt of perindopril | 4.00 | 8.00 | 4.00 | 8.00 |
| pregelatinized starch | 65.50 | 65,50 | 65.50 | 65.50 |
| sodium stearyl fumarate | 1.50 | 1.50 | 1.50 | 1.50 |
| Weight of the powder | 71.00 | 75.00 | 71.00 | 75.00 |

| Components of the granulate | | | | |
|---|---|---|---|---|
| amlodipine benzenesulfonate | 6.93 | 6.93 | 13.87 | 13.87 |
| microcrystalline cellulose | 95.47 | 95.47 | 88.53 | 88.53 |
| anhydrous dibasic calcium phosphate | 37.00 | 37.00 | 37.00 | 37.00 |
| Sodium starch glycolate | 2.00 | 2.00 | 2.00 | 2.00 |
| sodium stearyl fumarate | 1.60 | 1.60 | 1.60 | 1.60 |
| weight of the granulate | 143.00 | | | |
| *total weight of the blend* | 214.00 | 218.00 | 214.00 | 218.00 |

**Table 3. Qualitative and quantitative composition [mg] of amlodipine/ramipril - wet granulation**

| | | | | | |
|---|---|---|---|---|---|
| Component | AML/RAM 5/2,5 mg | AML/RAM 5/5 mg | AML/RAM 5/10mg | AML/RAM 10/5mg | AML/RAM 10/10 mg |

| Components of the powder | | | | | |
|---|---|---|---|---|---|
| Ramipril | 2.50 | 5.00 | 10.00 | 5.00 | 10.00 |
| Pregelatinized starch | 6.80 | 66.80 | 66.80 | 66.80 | 66,80 |
| Sodium stearyl fumarate | 0.70 | 0.70 | 0.70 | 0.70 | 0.70 |
| Weight of the powder | 70.00 | 72.50 | 77.50 | 72.50 | 77.50 |

| Components of the granulate | | | | | |
|---|---|---|---|---|---|
| Amlodipine benzenesulfonate | 6.93 | 6.93 | 6.93 | 13.87 | 13.87 |
| Microcrystalline cellulose | 109.47 | 109.47 | 109.47 | 102.53 | 102.53 |
| Anhydrous dibasic calcium phosphate, | 30.00 | 30.00 | 30.00 | 30.00 | 30.00 |
| Sodium starch glycolate | 6.40 | 6.40 | 6.40 | 6.40 | 6.40 |
| Sodium stearyl fumarate | 2.00 | 2.00 | 2.00 | 2.00 | 2.00 |
| Hydroxypropylmethylcel lulose | 7.20 | 7.20 | 7.20 | 7.20 | 7.20 |
| Weight of the granulate | 162 | | | | |
| *Total weight of the blend* | 232.00 | 234.50 | 239.50 | 234.50 | 239.50 |

### Example 1.

Batch quantities (based on Table 1) of amlodipine benzenesulfonate, microcrystalline cellulose, anhydrous dibasic calcium phosphate, sodium starch glycolate and sodium stearyl fumarate are sieved through a sieve. After mixing, the resulting blend is subjected to dry compaction. The resulting granulate of amlodipine was evaluated in terms of appearance, bulk density and uniformity of granules (average amlodipine benzenesulfonate content of 95 - 105%; RSD ≤ 5%). Then previously sieved components: ramipril, pregelatinized starch, and sodium stearyl fumarate were added as a powder blend to the granulate. The mixture was blended until a homogeneous and stable blend was obtained (the average amlodipine benzenesulfonate content of 95 - 105%; RSD ≤ 5%).

### Example 1A.

Analogously to Example 1, a stable composition of amlodipine and perindopril (based on Table 2) was obtained.

### Example 1B.

The following stable compositions were prepared analogously to Example 1 or 1A: amlodipine and ramipril: and amlodipine and perindopril, respectively, except that the granules of amlodipine were coated with a coating of hydroxypropylmethylcellulose and colloidal silica.

### Example 2.

Analogously to Example 1 a stable composition of ramipril and amlodipine (based on Table 3) was prepared, with the difference that amlodipine benzenesulfonate, microcrystalline cellulose, anhydrous dibasic calcium phosphate, and sodium stearyl fumarate were wet granulated with 10% aqueous solution of hydroxypropylmethylcellulose.

### Example 2A.

A stable composition of amlodipine and ramipril was prepared analogously to Example 2, except that the amlodipine granules were coated with a coating containing hydroxypropylmethylcellulose and colloidal silica,

### Example 3. Preparation of sachets containing a stable composition of an ACE inhibitor and a calcium channel blocker

A) Stick- type sachetes were filled with the composition obtained according to Example 1 in the form of the blend of the granulate of amlodipine and the powder containing ramipril.
B) Stick- type sachetes were filled with the composition obtained according to Example 2 in the form of the blend of the granulate of amlodipine and the powder containing ramipril.

### Example 4. Preparation of capsules containing stable composition of an ACE inhibitor and a calcium channel blocker

A) Hard gelatin capsules of size 1 were filled with the composition obtained according to Example 1 in the form of the blend of the granulate of amlodipine and the powder containing ramipril.
B) Hard gelatin capsules size 0 were filled with the composition obtained according to Example 2 in the form of the blend of the granulate of amlodipine and the powder containing ramipril.

### Example 5. Preparation of tablets containing stable composition of an ACE inhibitor and a calcium channel blocker.

The composition obtained according to Example 1A in the form of the blend of the granulate of amlodipine and the powder containing perindopril was compressed to a tablet using a high-speed tablet press Korsch type XL 100 to give tablets with the following parameters:
AML_PER dose 5 mg/4 mg:
   - mass: 214 mg
   - tablet diameter: 8 mm
   - hardness: 40-60 N
   - disintegration time: below 5 min
AML_PER dose 5 mg/8 mg:
   - mass: 218 mg
   - tablet diameter: 8 mm
   - hardness: 35 - 50 N
   - disintegration time: below 5 min

### Example 6.

Analogously to Examples 1-5, stable compositions and unit dosage forms were prepared for the combination of active substances ramipril and felodipine, felodipine and perindopril, trandolapril and felodipine, felodipine and quinapril, moexipril and felodipine, felodipine and fosinopril, enalapril and felodipine, amlodipine and perindopril, amlodipine and trandolapril, Amlodipine and quinapril, amlodipine and moexipril, amlodipine and fosinopril, amlodipine and enalapril, as well as capsules, sachets and tablets containing these compositions.

### Example 7. Stability of the capsules according to Example 4A, containing a composition according to Example 1.

Impurities of unit dosage form prepared according to Example 4A in the form of a capsule containing a pharmaceutical composition of ramipril and amlodipine obtained for amlodipine and ramipril doses 5mg/5mg; 5mg/10mg and 10mg/10mg, respectively, were determined using the method and the limits laid down in the relevant British Pharmacopoeia monographs for the impurity D and E of ramipril and experimentally determined on the basis of the stability tests in accordance with the guidelines of the International Conference on Harmonization Requirements for Medicines for impurity D of amlodipine.

**Table 4. Impurities of ramipril and amlodipine**

| | |
|---|---|
| Ramipril impurity D (ramipril diketopiperazine) | ethyl (2S)-2-[(3S,5aS,8aS,9aS)-3-methyl-1,4-dioxo-decahydro-1H-cyclopenta[e]pyrrolo[1,2a]pyrazin-2-yl]-4-phenylbutanoate |
| Ramipril impurity E (ramipril adiacid) | (2S,3aS,6aS)-1-[(2S)-2-{[(1S)-1-carboxy-3-phenylpropyl]amino}propanoyl]-octahydrocyclo-penta[b]pyrrole-2-carboxylic acid |
| Amlodipine impurity D | 3-ethyl,5-methyl ester 2-[(2-aminoethoxy)-methyl]-4-(2-chlorophenyl)-6-methylpyridine-3,5-dicarboxylate |

Stability of the pharmaceutical composition in the form of capsules containing amlodipine and ramipril in respective doses of 5 mg/5mg; 5mg/10mg and 10mg/10mg prepared according to Example 4A was determined by assessment of chemical purity using HPLC method at the start of experiment and after 3 and 6 months of storage at 40°C/75% relative humidity.

The results are shown in the following Tables 5, 6 and 7.

**Table 5 Chemical purity of amlodipine/ramipril 5 mg / 5 mg capsule by HPLC method**

| Requirements (limits of impurities) | Start | 3 months | 6 months |
|---|---|---|---|
| Amlodipine impurity D ≤ 0.5% | < 0.05% | 0.07% | 0.08% |
| Ramipril impurity D ≤ 5.0% | 0.16% | 2.5% | 3.9% |
| Ramipril impurity E ≤ 2.0% | < 0.05% | 0.17% | 0.20% |
| Max, unknown impurity ≤ 0.5% | < 0.1% | < 0.1% | 0.14% |
| Total impurities ≤ 7.0% | 0.16% | 2.7% | 4.3% |

**Table 6. Chemical purity of amlodipine/ramipril 5 mg/10 mg capsule by HPLC method**

| Requirements (limits of impurities) | Start | 3 months | 6 months |
|---|---|---|---|
| Amlodipine impurity D ≤ 0.5% | 0.05% | 0.10% | 0.11% |
| Ramipril impurity D ≤ 5.0% | 0.14% | 1.50% | 2.2% |
| Ramipril impurity E ≤ 2.0% | < 0.05% | 0.10% | 0.12% |
| Max, unknown impurity ≤ 0.5% | < 0.1% | < 0.1% | < 0.1% |
| total impurities ≤ 7.0% | 0.14% | 1.7% | 2.4% |

**Table 7. Chemical purity of amlodipine / ramipril 10 mg/10 mg capsule by HPLC method**

| Requirements (limits of impurities) | Start | 3 months | 6 months |
|---|---|---|---|
| Amlodipine impurity D ≤ 0.5% | 0.05% | 0.05% | 0.05% |
| Ramipril impurity D ≤ 5.0% | 0.15% | 1.30% | 2.3% |
| Ramipril impurity E ≤ 2.0% | < 0.05% | 0.05% | 0.12% |
| Max, unknown impurity ≤ 0.5% | < 0.1% | < 0.1% | < 0.1% |
| Total impurities ≤ 7.0% | 0.15% | 1.3% | 2.5% |

An increase of impurity D of ramipril and slight increase of impurities E of ramipril and D of amlodipine was observed. Percentage of detected impurities did not exceed the values indicated in the above mentioned monographs for pharmaceutical products and guidelines for medicinal substances.

### Example 8 Determination of pH of the composition

1.00 g of the compositions of Example 1 were accurately weighed to 10 ml flasks, then 9.00 g of Milli Q water was added, and the flasks were closed with stoppers. Two samples of each blend were weighed out. First sample was dissolved for 10 minutes in an ultrasonic bath and then for 10 minutes on a mechanical shaker. After that pH of the samples was measured. Second samples were mixed and the pH was measured. Results of pH measurements of the compositions of Example 1, containing amlodipine and ramipril in doses: 5 mg/2.5 mg; 5 mg/5 mg; 5 mg/10 mg; 10 mg/5mg; 10 mg/10 mg and pH measurements for powder a) containing 2.5 mg, 5 mg and 10 mg of ramipril are presented in Table 8.

**Table 8. pH values of compositions of Example 1 and powders a) containing ramipril.**

| | pH value sample 1 | pH value sample 2 |
|---|---|---|
| Composition of AML-RAM 5 mg /2.5 mg | 5.58 | 5.57 |
| Composition of AML-RAM 5 mg /5 mg | 5.52 | 5.51 |
| Composition of AML-RAM 5 mg /10 mg | 5.36 | 5.40 |
| Composition of AML-RAM 10 mg /5 mg | 5.37 | 5.35 |
| Composition of AML-RAM 10 mg/10 mg | 5.17 | 5.20 |
| RAM powder 2.5 mg | 4.99 | 5.01 |
| RAM powder 5 mg | 4.98 | 4.97 |
| RAM powder 10 mg | 4.97 | 4.95 |

### Example 9. Analysis of the compatibility of the active substances amlodipine benzenesulfonate and perindopril. tert-butylamine

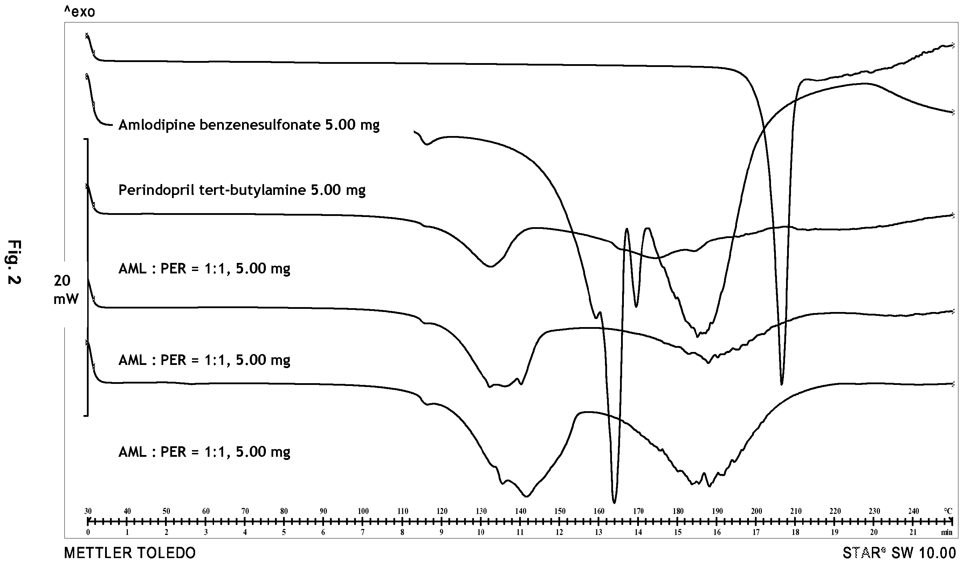

A DSC analysis was performed in an open system at a heating rate of 10°C per minute using a Mettler DSC Tolledo 1 Star System. Figure 2 shows the results of analysis of compatibility of amlodipine benzenesulfonate and perindopril tert-butylamine as a simple mixture of powders. The absence of a peak derived from melting of amlodipine benzenesulfonate in binary mixtures of amlodipine benzenesulfonate - perindopril tert-butylamine suggests incompatibility of these two substances.

The results of experiments presented in the examples show clearly that the composition and unit dosage form according to the invention containing incompatible active substances susceptible to degradation, manufactured in a simple and economically advantageous process according the invention are stable in short-and long-term storage conditions and meet chemical purity limits set for pharmaceutical preparations.

The advantage of inventive composition is no need for pH stabilizers or regulators.

Further advantage of the invention is that known excipients commonly used in the manufacture of solid dosage forms, that are described in pharmacopoeial monographs and thus ensure patient safety, are used in the composition. Another advantage of the composition of the invention is no need for physical separation of the active ingredients - while maintaining their stability.

The advantage of the process of the preparation of a composition according to the invention is the use of only non-labor intensive and simple operations of blending and compaction. An additional advantage of this process is that mechanical stress which contributes to the degradation of ACE inhibitors is minimized.

## Claims

1. A pharmaceutical composition in the form of a blend consisting of
a) a powder containing an ACE inhibitor wherein the ACE inhibitor is selected from ramipril, perindopril and pharmaceutically acceptable salts, hydrates, and solvates thereof, and
b) a granulate containing a calcium channel blocker wherein the calcium channel blocker is selected from amlodipine and pharmaceutically acceptable salts, hydrates, and solvates thereof,
wherein the powder a) and the granulate b) contain one or more excipients, and wherein the powder a) is not physically separated from the granulate b).

2. The composition according to claim 1, wherein the ACE inhibitor is tert-butylamine or arginine salt of perindopril, or ramipril in the form of free acid.

3. The composition according to any one of the preceding claims, wherein the percentage of the ACE inhibitor in the powder a) is from 2.5 to 14% by weight, with respect to the total weight of the powder a).

4. The composition according to any one of the preceding claims, wherein the calcium channel blocker is amlodipine benzenesulfonate.

5. The composition according to any one of the preceding claims, wherein the percentage of the calcium channel blocker in the granulate b) is from 3.5 to 11% by weight, with respect to the total weight of the granulate b).

6. The composition according to any one of the preceding claims, wherein the powder a) contains excipients selected from a filler and a glidant.

7. The composition according to claim 6, wherein the powder a) contains from 80 to 97% by weight of the filler, with respect to the total weight of the powder a).

8. The composition according to claim 6, wherein the powder a) contains from 0.5 to 2.5% by weight of the glidant, with respect to the total weight of the powder a).

9. The composition according to any one of the preceding claims, wherein the granulate b) contains excipients selected from a filler, binder, disintegrant and glidant.

10. The composition according to claim 9, wherein the granulate b) contains from 55 to 70% by weight of the filler, with respect to the total weight of the granulate b).

11. The composition according to claim 9, wherein the granulate b) contains from 15 to 30% by weight of the binder, with respect to the total weight of the granulate b).

12. The composition according to claim 9, wherein the granulate b) contains from 0.8 to 4.3% by weight of the disintegrant, with respect to the total weight of the granulate b).

13. The composition according to claim 9, wherein the granulate b) contains from 0.8 to 1.5% by weight of the glidant, with respect to the total weight of the granulate b).

14. A unit dosage form comprising a composition as defined any one of the claims 1 to 11 selected from a capsule, a sachet, a stick, a tablet and an ampoule.

15. The unit dosage form according to claim 14, containing the ACE inhibitor in the amount of 0.9 to 5.0% by weight and the calcium channel blocker in the amount of 2.6 to 7.1% by weight with respect to the total weight of the composition in the dosage unit form.

## Patentansprüche

1. Pharmazeutische Zusammensetzung in Form einer Mischung bestehend aus
a) einem Pulver, das einen ACE-Inhibitor enthält, wobei der ACE-Inhibitor aus Ramipril, Perindopril, und deren pharmazeutisch akzeptable Salze, Solvate und Hydrate ausgewählt ist, und
b) einem Granulat, das einen Calciumkanalblocker enthält, wobei der Calciumkanalblocker aus Amlodipin und deren pharmazeutisch akzeptable Salze, Solvate und Hydrate ausgewählt ist, und
wobei das Pulver a) und der Granulat b) einen oder mehrere Hilfsstoffe enthalten, und das Pulver a) nicht physikalisch von dem Granulat b) getrennt ist.

2. Zusammensetzung nach Anspruch 1, wobei der ACE-Inhibitor Perindopril in Form des tert-Butylamin- oder Arginin-Salzes oder Ramipril in Form der freien Säure ist.

3. Zusammensetzung nach einem der vorhergehenden Ansprüche, wobei der Prozentanteil des ACE-Inhibitors im Pulver a) 2,5 bis 14 Gew.-%, bezogen auf das Gesamtgewicht des Pulvers a) beträgt.

4. Zusammensetzung nach einem der vorhergehenden Ansprüche, wobei der Calciumkanalblocker Amlodipinbenzolsulfonat ist.

5. Zusammensetzung nach einem der vorhergehenden Ansprüche, wobei der Prozentanteil des Calciumkanalblockers im Granulat b) 3,5 bis 11 Gew.-%, bezogen auf das Gesamtgewicht des Granulats b) beträgt.

6. Zusammensetzung nach einem der vorhergehenden Ansprüche, wobei das Pulver a) Hilfsstoffe enthält, die aus einem Füllstoff und einem Gleitmittel ausgewählt sind.

7. Zusammensetzung nach Anspruch 6, wobei das Pulver a) 80 bis 97 Gew.-% eines Füllstoffs, bezogen auf das Gesamtgewicht des Pulvers a), enthält.

8. Zusammensetzung nach Anspruch 6, wobei das Pulver a) 0,5 bis 2,5 Gew.-% eines Gleitmittels, bezogen auf das Gesamtgewicht des Pulvers a), enthält.

9. Zusammensetzung nach einem der vorhergehenden Ansprüche, wobei der Granulat b) Hilfsstoffe enthält, die aus einem Füllstoff, einem Bindemittel, einem Zerfallsmittel und einem Gleitmittel ausgewählt sind.

10. Zusammensetzung nach Anspruch 9, wobei das Granulat b) 55 bis 70 Gew.-% eines Füllstoffs, bezogen auf das Gesamtgewicht des Granulats b), enthält.

11. Zusammensetzung nach Anspruch 9, wobei das Granulat b) 15 bis 30 Gew.-% des Bindemittels, bezogen auf das Gesamtgewicht des Granulats b), enthält.

12. Zusammensetzung nach Anspruch 9, wobei das Granulat b) 0,8 bis 4,3 Gew.-% des Zerfallsmittel, bezogen auf das Gesamtgewicht des Granulats b), enthält.

13. Zusammensetzung nach Anspruch 9, wobei das Granulat b) 0,8 bis 1,5 Gew.-% des Gleitmittels, bezogen auf das Gesamtgewicht des Granulats b), enthält.

14. Einheitsdosierungsform, umfassend eine Zusammensetzung nach einem der Ansprüche 1 bis 13, ausgewählt aus einer Kapsel, einem Sachet, einem Stick, einer Tablette und einer Ampulle.

15. Einheitsdosierungsform nach Anspruch 14, enthaltend einen ACE-Inhibitor in einer Menge von 0,9 bis 5,0 Gew.-% und einen Calciumkanalblocker in einer Menge von 2,6 bis 7,1 Gew.-%, bezogen auf das Gesamtgewicht der Zusammensetzung in die Einheitsdosierungsform.

## Revendications

1. Une composition pharmaceutique sous la forme d'un mélange consistant en
a) une poudre contenant un inhibiteur de l'ECA, l'inhibiteur de l'ECA etant choisi dans le groupe consistant en perindopril, ramipril, et pharmaceutiquement acceptables sels, solvates et hydrates de ceux-ci, et
b) un granulé contenant un bloqueur des canaux calciques, le bloqueur des canaux calciques etant choisi parmi l'amlodipine et les sels pharmaceutiquement acceptables, solvates et hydrates de ceux-ci,
dans laquelle la poudre a) et le granulé b) contiennent un ou plusieurs excipients,
et dans laquelle la poudre a) et le granulé b) ne sont pas physiquement séparés l'un de l'autre.

2. La composition selon la revendication 1, dans laquelle l'inhibiteur de l'ECA est le sel de la tert-butylamine ou de l'arginine du perindopril, ou le ramipril sous forme d'acide libre.

3. La composition selon l'une quelconque des revendications précédentes, dans laquelle le pourcentage d'inhibiteur de l'ECA dans la poudre a) est de 2,5 à 14% en poids, par rapport au poids total de la poudre a).

4. La composition selon l'une quelconque des revendications précédentes, dans laquelle le bloqueur des canaux calciques est le benzènesulfonate d'amlodipine,

5. La composition selon l'une quelconque des revendications précédentes, dans laquelle le pourcentage des canaux calciques dans le granulé b) est de 3,5 à 11 % en poids, par rapport au poids total du granulé b).

6. La composition selon l'une quelconque des revendications précédentes, dans laquelle la poudre a) contient des excipients choisis parmi un diluent et un lubrifiant.

7. La composition selon la revendication 6, dans laquelle la poudre a) contient de 80 à 97% en poids du diluant, par rapport au poids total de la poudre a).

8. La composition selon la revendication 6, dans laquelle la poudre a) contient de 0,5 à 2,5% en poids du lubrifiant, par rapport au poids total de la poudre a).

9. La composition selon l'une quelconque des revendications précédentes, dans laquelle le granulé b) contient des excipients choisis parmi du diluant, du liant, du désintégrant et du lubrifiant.

10. La composition selon la revendication 9, dans laquelle le granulé b) contient de 55 à 70% en poids du diluant, par rapport au poids total du granulé b).

11. La composition selon la revendication 9, dans laquelle le granulé b) contient de 15 à 30% en poids du diluant, du liant, par rapport au poids total du granulé b).

12. La composition selon la revendication 9, dans laquelle le granulé b) contient de 0,8 à 4,3% en poids du désintégrant, par rapport au poids total du granulé b).

13. La composition selon la revendication 9, dans laquelle le granulé b) contient de 0,8 à 1,5% en poids du désintégrant, par rapport au poids total du granulé b).

14. Une forme de dosage unitaire comprenant une composition selon l'une quelconque des revendications 1 à 13 choisie parmi une capsule, un sachete, un bâton, un comprimé et une ampoule.

15. La forme de dosage unitaire selon la revendication 14, contenant un inhibiteur de l'ECA en quantité de 0,9 à 5,0% en poids et un inhibiteur des canaux calciques en quantité de 2,6 à 7,1% en poids par rapport au poids total de la composition dans la forme de dosage unitaire.
